# EUROPÄISCHE PATENTSCHRIFT

(11) **EP 1 471 967 B1**
(45) Veröffentlichungstag und Bekanntmachung des Hinweises auf die Patenterteilung: **21.06.2006**
(21) Anmeldenummer: 02716035.7
(22) Anmeldetag: 07.02.2002
(51) Int. Cl.: A61M 25/01

(54) **AUSLENKVORRICHTUNG FÜR KATHETER**
DISPLACEMENT DEVICE FOR A CATHETER
DISPOSITIF DE DEPLACEMENT POUR CATHETER

(43) Veröffentlichungstag der Anmeldung: 03.11.2004
(73) Patentinhaber: Carag AG, 6340 Baar (CH)
(72) Erfinder: THOMMEN, Daniel, 6300 Zug (CH)
(74) Vertreter: Clerc, Natalia
(86) Internationale Anmeldenummer: PCT/CH2002/000074
(87) Internationale Veröffentlichungsnummer: WO 2003/066149

(56) Entgegenhaltungen:
- WO-A-01/78825
- WO-A-96/32980
- WO-A-97/13542
- DE-A- 4 333 090

## Beschreibung

Die vorliegende Erfindung betrifft einen Katheter, insbesondere Herzkatheter, ausgerüstet mit einer Vorrichtung zur einstellbaren Auslenkung des distalen Endabschnittes eines flexiblen Katheterschlauches.

Das Einführen eines Katheters durch Gefässe (Venen) zum Herz eines Patienten, aus der Leisten- oder Oberschenkelzone ist eine übliche Technik, welche weltweit angewendet wird. Das Vorgehen ist problemlos bis in das Herz (z.B. Plazierung in den linken Vorhof durch ein Atrium Septum Defect). Schwieriger, wenn nicht unmöglich, wird die Sache, wenn ein eingeführter Katheter orientiert werden muss; um z.B. das distale Ende des Katheters von einer Herzwand zu entfernen, um mehr Freiheit zur Platzierung eines Implantats zu bekommen. Operationen in den Herzvorkammern, z.B. bei einem Atrium Septum Defect, werden deshalb meistens durch klassische Chirurgiemethoden (von aussen) durchgeführt.

Aus WO 96/32980 ist ein Katheter mit einem vorgebogenem Katheterschlauch bekannt, welcher an seinem distalen Ende gebogen ausgebildet ist. In diesem Katheterschlauch kann ein Draht hin und her geschoben werden. Wird der Schlauch bis in den Bereich des distalen Endes des Schlauches geschoben, wird die Krümmung des Schlauchs mindestens teilweise aufgehoben. Wird der Draht wieder zurückgezogen, krümmt sich der Schlauch in seine vorgegebene Stellung.

Aufgabe der vorliegenden Erfindung war es somit, Mittel zu schaffen, welche das Einführen von Kathetern, insbesondere Herzkathetern, auch unter schwierigen Bedingungen ermöglichen, um so gewisse Eingriffe auch mittels minimalinvasiver Chirurgie zu erlauben.

Dass diese Aufgabe zu lösen wäre, wenn der distale Endabschnitt eines Katheterschlauches von aussen gesteuert ausgelenkt werden könnte, liegt auf der Hand. Eine Lösung mit vertretbarem Aufwand war jedoch nicht in Sicht.

Gemäss der vorliegenden Erfindung ist die Aufgabe mit einer Vorrichtung mit den Merkmalen gemäss dem kennzeichnenden Teil von Anspruch 1 bzw. mit einem gemäss dem kennzeichnenden Teil von Anspruch 2 auf überraschend einfache Weise lösbar.

Ein Ausführungsbeispiel des Erfindungsgegenstandes wird nachstehend anhand der Zeichnung noch etwas näher erläutert. Es zeigt:
- Fig. 1: rein schematisch, einen mit einer erfindungsgemässen Auslenkvorrichtung ausgerüsteten Katheter in Neutralstellung, und
- Fig. 2: den Katheter nach Fig. 1 mit um einen einstellbaren Winkel ausgelenktem Endabschnitt.

Die Zeichnung zeigt einen Katheterschlauch 1 aus flexiblem Material mit einem durch den Schlauch geführten Führungsdraht 2 aus federelastischem Material (z.B. Stahl), dessen vor dem distalen Ende 1' des Schlauches liegender Endabschnitt 2' spiralförmig geformt ist. Der Draht 2 lässt sich im Schlauch 1 gleitend verschieben (durch Zug auf das untere Ende des Drahtes 2).

Im distalen Ende 1' des Schlauches 1 ist ein steifes Rohrstück 3 (Hülse) angeordnet.

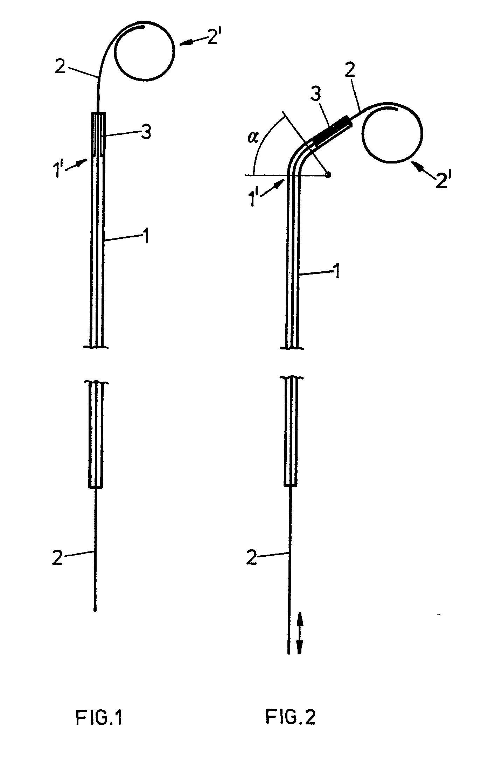

Der Katheter als Herzkatheter, d.h. Katheterschlauch 1 mit Führungsdraht 2, kann in der in Figur 1 gezeigten Konfiguration durch eine Vene mehr oder weniger geradeaus bis zum Herzen eingeführt werden. Wenn dort der weitere Weg unter einem vorbestimmten Winkel weiterführt, kann durch Zug auf das hintere Ende des Drahtes die Spirale 2' mehr oder weniger stark in den Schlauch 1 gezogen werden, was wegen der Federspannung zur gesteuerten Auslenkung (um einen einstellbaren Winkel α) des distalen Endabschnittes 1' des Schlauches 1 führt, und letzterer kann problemlos weitergeschoben werden. Die gewünschte Einstellung der Auslenkung α wird durch ein in das Schlauchende eingesetztes steifes Rohrstück 3, z.B. in Form einer Metallhülse, erleichtert.

Die Einstellung des Auslenkungswinkels erfolgt durch mehr oder weniger starkes Einziehen der Drahtspirale 2' in den Schlauch.

## Patentansprüche

1. Katheter, insbesondere Herzkatheter, mit einem flexiblen Katheterschlauch und einer Vorrichtung zur einstellbaren Auslenkung des distalen Endabschnittes des Katheterschlauches wobei die Auslenkvorrichtung durch einen Führungsdraht aus federelastischem Material gebildet ist, welcher Führungsdraht dazu vorgesehen ist, gleitend durch den Katheterschlauch in eine Neutral- oder Ausgangslage geführt zu werden, wobei bei mehr oder weniger starkem Einziehen des Drahtes aus seiner Ausgangslage der Endabschnitt des flexiblen Katheterschlauchs ausgelenkt wird, **dadurch gekennzeichnet, dass** der Führungsdraht einen spiralförmig geformten Endabschnitt aufweist, wobei in der Neutral- oder Ausgangslage das spiralförmig ausgebildete Drahtende direkt vor dem genannten äusseren Ende des Katheterschlauches liegt, derart, dass beim Einziehen des Drahtes aus seiner Ausgangslage in den flexiblen Katheterschlauch der Endabschnitt des letzteren unter dem Einfluss der Federkraft des sich dabei zwangsläufig öffnenden spiralförmigen Drahtendes um einen einstellbaren Winkel ausgelenkt wird.

2. Katheter nach Anspruch 2, **dadurch gekennzeichnet, dass** am distalen Ende des Katheterschlauches ein steifes Rohrstück angeordnet ist.

3. Katheter nach Anspruch 3, **dadurch gekennzeichnet, dass** das steife Rohrstück eine Metallhülse ist.

## Claims

1. Catheter, in particular a cardiac catheter, with a flexible catheter tube and a device for adjustably displacing the distal end portion of the catheter tube, wherein the displacement device is formed by a guide wire made out of spring-elastic material, which guide wire is foreseen to be guided slidably through the catheter tube into a neutral position or starting position, whereby the wire is drawn to a greater or lesser extent from its starting position, the end portion of the flexible catheter tube will be displaced, **characterized in that** the guide wire has a spirally shaped end portion, whereby in the neutral position or starting position of the spirally shaped end portion lies directly in front of said outer end of the catheter tube in such a way that when the wire is drawn from its starting point into the flexible catheter tube, the end portion of the latter is displaced by an adjustable angle under the action of the spring force of the spirally shaped wire end, which necessarily opens out.

2. Catheter according to claim 1, **characterized in that** a stiff tubular piece is arranged at the distal end of the catheter tube.

3. Catheter according to claim 2, **characterized in that** the stiff tubular piece is a metal sleeve.

## Revendications

1. Cathéter, en particulier cathéter cardiaque, avec un tuyau de cathéter flexible et un dispositif pour la déviation réglable de la section d'extrémité distale d'un tuyau de cathéter, le dispositif de déviation étant constitué d'un fil de guidage en matériau élastique, qui est prévu pour être guidé par glissement à travers le tuyau du cathéter en position neutre ou position de départ, dans lequel, par traction plus ou moins forte du fil, la section d'extrémité du tuyau de cathéter flexible est déviée de sa position de départ, **caractérisé en ce que** le fil de guidage présente une section d'extrémité de forme spiralée, l'extrémité du fil de forme spiralée se situant, en position neutre ou en position de départ, directement devant l'extrémité externe citée du tuyau du cathéter de sorte que, lorsque l'on tire le fil de sa position de départ dans le tuyau de cathéter flexible, la section d'extrémité de ce dernier soit déviée d'un angle réglable sous l'action de la force élastique de l'extrémité du fil spiralée s'ouvrant en l'occurrence à force.

2. Cathéter selon la revendication 1, **caractérisé en ce qu'**une pièce tubulaire rigide est aménagée sur l'extrémité distale du tuyau de cathéter.

3. Cathéter selon la revendication 2, **caractérisé en ce que** la pièce tubulaire rigide est une douille métallique.
